# EUROPEAN PATENT SPECIFICATION

(11) **EP 0 298 636 B1**
(45) Date of publication and mention of the grant of the patent: **20.03.1996**
(21) Application number: 88305799.4
(22) Date of filing: 23.06.1988
(51) Int. Cl.: C07C 269/04

(54) **Process for the production of carbamates**
Verfahren zur Herstellung von Carbaminsäureestern Verfahren zur Herstellung von Carbaminsäureester
Procédé de preparation de carbamates

(30) Priority: 25.06.1987 GB 8714873; 25.06.1987 GB 8714872; 25.06.1987 GB 8714874
(43) Date of publication of application: 11.01.1989
(73) Proprietor: BP Chemicals Limited, London EC2M 7BA (GB)
(72) Inventor: A'Court, Richard, Hull HU12 8DS (GB); Hamlin, John Edward, Hull HU12 8DS (GB); Fox, William Joseph, Blackheath London S.E.3 (GB); O'Connor, Sean Patrick, Hull HU12 8DS (GB)
(74) Representative: Barlow, Michael Thomas

(56) References cited:
- EP-A- 0 152 240
- FR-A- 2 355 812
- GB-A- 1 124 646
- GB-A- 1 338 407
- GB-A- 2 091 730
- US-A- 4 236 020

## Description

The present invention relates a process for the production of carbamates.

Carbamates are useful in agrochemicals, resins, plasticisers and monomers. A number of therapeutic applications include use as antipyretics, diuretics and antiseptics. A widely used method for the production of carbamates is the reaction of an alkyl isocyanate with an alcohol. A disadvantage of this method is that alkyl isocyanates are highly toxic materials. Other reported methods for producing carbamates are disclosed in Japan Kokai 77, 14745 wherein NaOMe/MeOH, PhNH₂ and (MeO)₂ CO are reacted to produce PhNHCO₂Me, Japan Kokai 79, 163528 which discloses a similar reaction using NaOMe, piperidine, imidazole, carbonyldiimidazole, N-methylpyrolidone or morpholine, US-A-4258683 wherein aromatic amines are reacted with organic carbonates in the presence of zinc and stannous salts, and US-A-4268684 wherein there is disclosed a similar reaction to that of US-A-4268683 except that cobaltous salts are used instead of zinc and stannous salts.

Carbamates have also been used as additives to fuels.

A specific problem thought to involve deposit formation in the combustion chamber is that of octane requirement increase (generally abbreviated to ORI). The problem of ORI is addressed by US Patent No. 4,236,020, the solution to the problem according to the aforesaid US Patent being to add to the fuel a poly(oxyalkylene)carbamate soluble in a hydrocarbon fuel boiling in the gasoline range. The carbamates of USP 4,236,020 comprise a hydrocarbyloxy-terminated poly(oxyalkylene) chain of at least 5 oxyalkylene units containing 2 to 5 carbon atoms per oxyalkylene unit bonded through an oxycarbonyl group to a nitrogen atom of ethylenediamine. Preferred carbamates are those described by the general formula: 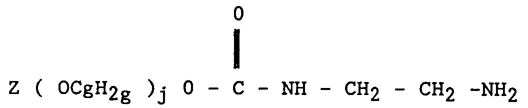
in which g is an integer 2 to 5, j is an integer such that the molecular weight of the compound is in the range of about 1,200 to about 5,000, Z is a hydrocarbyl of 1 to 30 carbon atoms and sufficient of the oxyalkylene units in the compounds are other than ethylene to render the compounds soluble in hydrocarbon fuels boiling in the gasoline range. The only method disclosed for preparing the carbamates is the reaction of a suitable capped polyether alcohol with phosgene to form a chloroformate followed by reaction of the chloroformate with ethylene diamine to form the active carbamate. A problem associated with this route to carbamates is the use of phosgene which not only is highly toxic but also can lead to products contaminated with chlorine.

We have now found an improved process for the production of carbamates wherein a strong organic base which is an amidine or a Lewis base and an epoxide is used as the catalyst.

Accordingly, in a first aspect, the present invention provides a process for the production of a carbamate having the formula: 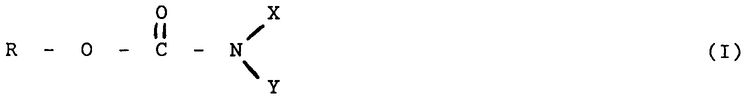
wherein X and Y are independently either hydrogen, a hydrocarbyl group or a hetero-substituted hydrocarbyl group or the group of formula: 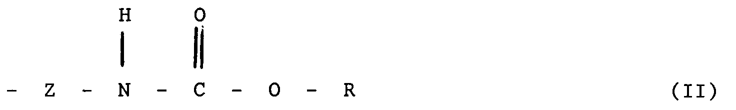
wherein Z is either a divalent hydrocarbyl or substituted hydrocarbyl group or a group of the formula:

[ (alkylene)ₘ(NH)ₙ(alkylene)ₘ ] (III)

in which n = 0 to 4 and m = 1 to 4, and
R is a hydrocarbyl or substituted hydrocarbyl group, provided that if either one of X or Y is the group of formula (II), the other of X or Y is hydrogen,
which process comprises reacting a compound of the formula: 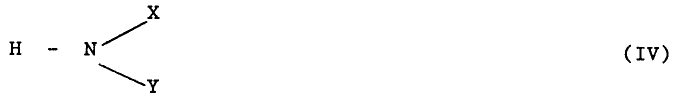
wherein X and Y are independently either hydrogen, a hydrocarbyl group, or a hetero-substituted hydrocarbyl group with a dihydrocarbyl carbonate having the formula: 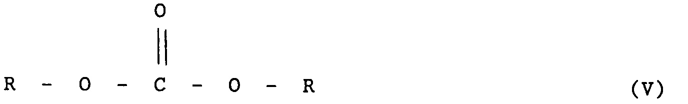
wherein independently R is as defined above in the presence as catalyst of a strong organic base which is an amidine or a Lewis base and an epoxide and in the proportions necessary to produce either a mono-carbamate or a bis-carbamate.

Preferably at least one mole of the compound of formula (IV) per mole of the dihydrocarbyl carbonate of formula (V) is employed.

The compound of formula (IV) may be either ammonia (X = Y = H), a primary amine (either of X or Y = H) or a secondary amine (neither of X or Y = H). The amine may suitably be either a monoamine or a polyamine. X and Y may be independently either a hydrocarbyl group or a hetero-substituted hydrocarbyl group. Suitably the hydrocarbyl group may be an aliphatic hydrocarbyl group, of which alkyl groups are preferred. Examples of suitable hydrocarbyl groups include methyl, ethyl, propyl or butyl groups. Alternatively, X and Y may be independently a hetero-substituted hydrocarbyl group. Suitably, the hetero-substituted hydrocarbyl group may be an aliphatic hydrocarbyl group substituted by nitrogen or oxygen, preferably a group of the formula -P-Q, wherein P is an alkylene or polyalkylene group, for example a C₁ to C₄ alkylene group or a poly- C₁ to C₄ alkylene group, and Q is either an NH₂, -OH or a heterocyclic group, for example morpholine or piperazine, or a substituted heterocyclic group, for example pipecoline. Examples of suitable compounds (IV) wherein X and Y are independently hetero-substituted hydrocarbyl groups include alkanolamines, for example ethanolamine, and compounds of formula (IV) wherein:-
(i) X = H and Y = CH₂CH₂NH₂
(ii) X = H and 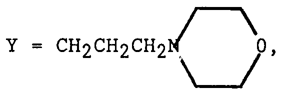 and
(iii) X = H and 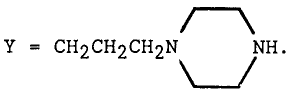
Finally, the compound (IV) in which X and Y are independently hetero-substituted hydrocarbyl groups, may be an alkylene polyamine or a polyalkylene polyamine, suitably wherein the alkylene group is a C₁-C₄ alkylene group, for example ethylene diamine, diethylene triamine or triethylene tetramine.

In the dihydrocarbyl carbonate of formula (V) the groups R are independently hydrocarbyl groups which may suitably be alkyl groups, preferably C₁ to C₄ alkyl groups, more preferably methyl groups. Alternatively, the dihydrocarbyl carbonate of formula (V) may be a cyclic carbonate, for example ethylene carbonate or propylene carbonate.

The process may be operated in the presence or absence of an added solvent. Suitable solvents include liquid hydrocarbon solvents, for example the mixed aromatic solvent designated A260 commercially available from BP Chemicals Limited.

The catalyst is a strong organic base which is an amidine or a Lewis base and an epoxide. A preferred strong organic base is an amidine. By the term amidine is meant a compound containing the grouping: 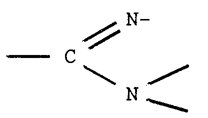
wherein the free valencies on the nitrogen atoms are attached to either carbon atoms or hydrogen and the free valency on the carbon atom is attached to either another carbon atom or nitrogen. In the case that the free valency on the nitrogen is attached to nitrogen the amidine is a guanidine.

A preferred class of amidine is the cyclic amidines. Cyclic amidines are defined as those amidines wherein at least one of the nitrogen atoms is part of an alicyclic or heterocyclic substituted or unsubstituted hydrocarbyl ring. In the case where the amidine is a guanidine then any two or the three nitrogen atoms may be in the same or different rings. Those nitrogen atoms which are not part of any said ring may form part of a substituted or unsubstituted hydrocarbyl group.

A preferred class of cyclic amidine is that in which the amidine group can form part of a fused ring system containing 6 and 5 membered rings or 6 and 7 membered rings or two six membered rings, as for example in 1,5-diazabicyclo [4.3.0] non-5-ene (DBN) which has the formula 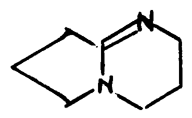
or 1,8-diazabicyclo [5.4.0] undec-7-ene (DBU) of the formula 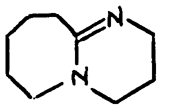
or 1,5,7-triazabicyclo [4.4.0] dec-5-ene (TBD) of formula 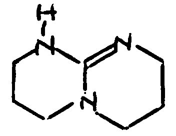
The amidine may be supported on a suitable support. This may be accomplished by deposition of the amidine on a support or by chemical bonding of the amidine to a suitable support. Suitable supported amidine catalysts are described in, for example, our EP-A-0168167.

As an alternative to an amidine, the strong base may comprise a Lewis base and an epoxide. The term Lewis base is generally understood to mean a compound containing an unshared pair of electrons capable of sharing with an acid. The terms Lewis base and amidine are not therefore mutually exclusive. The Lewis base may suitably be an organic compound containing trivalent nitrogen or phosphorus, for example an amine or a phosphine. The epoxide may suitably be a substituted or unsubstituted C₂ to C₈ alkylene oxide, preferably either ethylene oxide, propylene oxide or butylene oxide.

As regards the reaction conditions, the process may suitably be operated at a temperature in the range from 0 to 100°C, more preferably from 15 to 75°C, most preferably from 20 to 25°C, and the pressure may be either atmospheric or superatmospheric, for example from 1 to 10 bar.

The process may be operated batchwise or continuously, preferably continuously.

An advantage of using the process of the present invention for the production of carbamates is that it avoids the use of phosgene and its associated disadvantages.

The group R in the carbamate of formula (I) can be exchanged for a group R¹ by a process which comprises reacting the carbamate of formula (I) with a compound of the formula:

R¹OH (VI)

wherein R¹ is a hydrocarbyl or hetero-substituted hydrocarbyl group different from R, in the presence as catalyst of either a strong organic base or a tetrahydrocarbyl titanate.

The group R¹ in the compound of formula (VI) may suitably be any of the groups R as defined for the carbamate of formula (I), provided it is not identical to the group R. Hydrocarbyl groups substituted with nitrogen and/or oxygen, for example hydrocarbyl polyether groups, may be used.

In a preferred embodiment of the present invention there is provided a process for the production of a carbamate suitable for use as a detergent additive to internal combustion engine fuels wherein the group R¹ in the compound of formula (VI) is a hydrocarbyl or hetero-substituted hydrocarbyl group of a molecular weight and composition such as to impart fuel solubility.

A preferred compound of formula (VI) for this purpose is a polyalkyene glycol (PAG) formed by the reaction of a hydroxylic compound, which may be either an alcohol, or a phenol with an alkylene oxide, and may suitably have a molecular weight in the range from about 500 to 10,000, preferably from 1200 to 5000. PAGs are more fully described in the aforesaid US Patent No. 4,236,020, the disclosure of which in respect of suitable PAGs is incorporated by reference herein. The PAG should contain sufficient oxyalkylene units other than ethyleneoxy to effect solubility in internal combustion engine fuels. A particularly suitable compound of formula (VI) for use in the process of the invention is a polyoxyalkylene glycol obtained by the reaction of p-dodecylphenol with butylene oxide and having a molecular weight of about 2,000, which material is commercially available as BREOX (RTM) PC 1362 from Hythe Chemicals Limited. Another preferred class of compound of formula (VI) suitable for use in the production of detergent additives is polyalkylene glycols produced by the hydroxyalkylation, suitably by reaction with alkylene oxides, of amines, for example ethylene diamine or aminopropylmorpholine.

As catalyst for the exchange reaction there is used either a strong organic base or a tetrahydrocarbyltitanate. Suitable strong organic bases are those as hereinbefore described in relation to the preparation of carbamates of the formula (I). The same strong organic base or a different strong organic base, preferably the same, may be used in the exchange reaction as in the preparation of the carbamate of formula (I). Suitably the tetrahydrocarbyltitanate may be a tetraalkyltitanate. Suitably the alkyl group of the tetraalkyltitanate may be a C₁ to C₄ alkyl group. An example of a suitable tetraalkyltitanate is tetraisopropyltitanate.

It is preferred to react the compound of formula (I) with the compound of formula (VI) in the presence of a suitable solvent. Suitably the solvent may be a hydrocarbon solvent, for example the mixed aromatic hydrocarbon solvent identified as A260 which is commercially available from BP Chemicals Limited.

As regards the reaction conditions for reacting the compound of formula (I) with the compound of formula (VI), the temperature may suitably be elevated, preferably in the range from 100 to 300°C, and the pressure may be either atmospheric or superatmospheric.

A particular advantage of the process claimed for the production of detergent additives is that it provides a chlorine-free product, in contrast to prior art processes, such as that of US Patent No. 4,236,020.

An internal combustion engine fuel concentrate composition comprises as a first component from 1 to 95% by weight of a carbamate of the formula (I) wherein the group R is a hydrocarbyl or substituted hydrocarbyl group of a molecular weight and composition such as to impart fuel solubility as produced by a process as hereinbefore described and as a second component a fuel compatible solvent therefor.

The fuel compatible solvent for the compound of the formula (I) may suitably be an internal combustion engine fuel.

Preferably the carbamate is a mono-carbamate.

An internal combustion engine fuel composition comprises a major proportion of an internal combustion engine fuel and a minor proportion of the concentrate composition as hereinbefore described.

The amount of the concentrate composition present in the fuel composition may suitably be such as to provide a concentration of the compound of formula (I) in the fuel composition in the range from 10 to 10,000 p.p.m. by weight.

The internal combustion engine fuel is preferably a fuel boiling in the gasoline range. The fuel composition may incorporate additives conventionally employed in fuels compositions. Such additives may be incorporated either into the fuel concentrate or directly into the fuel composition.

The invention will now be further illustrated by reference to the following Examples.

### Preparation of monocarbamate of the formula (I)

### Example 1

Dimethylcarbonate (9.0g) and n-butylamine (7.3g) were mixed with TBD (0.05g) and allowed to stand at room temperature. After 6 h a sample of the liquid product was shown, by gc/ms, to be methyl N-(n-butyl) carbamate (95% yield).

### Example 2

Dimethylcarbonate (9.0g) and n-propylamine (5.9g) were mixed with TBD (0.05g) according to Example 1. The product was shown by gc/ms to be methyl N-(n-propyl) carbamate (93% yield).

### Example 3

Dimethylcarbonate (9.0g) and ethanolamine (4.7g) were mixed with TBD (0.05g) according to Example 1. The product was shown by gc/ms to be methyl N-(2-hydroxyethyl) carbamate (86% yield).

### Example 4

Example 1 was repeated replacing the dimethylcarbonate with diethylcarbonate (9.2g). The liquid product was shown by gc/ms to be ethyl N-(n-butyl)carbamate. (92% yield).

### Comparison Test 1

Example 1 was repeated in the absence of TBD. Only starting material was recovered.

### Comparison Test 2

Example 1 was repeated replacing the TBD with triethylamine (0.05g). The liquid product was shown by gc/ms to be methyl N-(-n-butyl) carbamate (6% yield).

### Example 5

Example 1 was repeated replacing TBD with DBU. The liquid product was shown by gc/ms to be methyl N-(n-butyl) carbamate (65% yield).

### Example 6

Example 1 was repeated replacing TBD with DBN. The liquid product was shown by gc/ms to be methyl N-(n-butyl) carbamate (30% yield).

### Example 7

A mixture of ethanolamine (50.6 g), dimethylcarbonate (74.8 g), TBD (1.0 g) and m-xylene (10 ml) was refluxed for 3 hours (ca. 80°C). The mixture was evaporated under reduced pressure giving the product as a clear liquid (84 g). NMR analysis indicated the product to be methyl [N-(2-hydroxyethyl)] carbamate.

### Example 8

A mixture of N-(2-aminoethyl) piperazine (28 g), dimethylcarbonate (20 g) and TBD (1.0 g) was left to stand open to the atmosphere for 120 hours. The mixture was evaporated under reduced pressure giving the product (38.75 g) identified by NMR to be methyl N-[2-(N¹-piperazino)ethyl] carbamate.

### Example 9

A mixture of N-(3-aminopropyl) pipecoline (37.1 g), dimethylcarbonate (23.0 g) and TBD (1.0 g) was allowed to stand open to the atmosphere for 120 hours. The mixture was evaporated under reduced pressure giving the product (46.1 g), identified by NMR to be methyl N-[3-(N¹-pipecolino)propyl] carbamate.

### Example 10

- X = H, 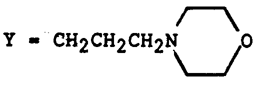
in formula (I).

### Step (A)

A mixture of N-(3-aminopropyl) morpholine (57.1 g), dimethyl carbonate (42 g) and TBD (1.0 g) was refluxed for 6 hours with a nitrogen sparge. The resultant mixture was evaporated under reduced pressure to give the product as a clear yellow liquid (74 g). NMR analysis indicated the product to be methyl N-[3-(N¹-morpholino) propyl] carbamate.

### Step (B)

The intermediate carbamate from Step A (16.5 g), BREOX PC 1362 (114 g) and TBD (1.0 g) were stirred at 150°C for 3.5 hours with a nitrogen sparge. The mixture was cooled, treated with toluene (50 ml) and magnesium sulphate (1.0 g) and allowed to stand. The mixture was then filtered and evaporated under reduced pressure to give the product as a clear page yellow liquid.
Analysis: % Nitrogen found = 1.46

### Example 11

- X = H, 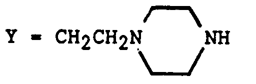
in the formula (I).

### Step (A)

A mixture of N-(2-aminoethyl)piperazine (20 g), dimethylcarbonate (33.6 g) and DBU (0.2 g) was stirred at 80-120°C for 2 hours with a nitrogen sparge. The mixture was cooled and filtered to give the product as a clear orange-yellow liquid. NMR analysis indicated the product to be methyl N-[2-(N¹-piperazino) ethyl] carbamate.

### Step (B)

The intermediate carbamate product from Step A (10.8 g, containing DBU catalyst carried through from Step A) and BREOX PC 1362 (136 g) were stirred at 120 - 140°C for 3 hours with a nitrogen sparge. The product was cooled and filtered to give a clear yellow liquid.
Analysis: % Nitrogen = 1.10.

### Example 12

A mixture of methyl N-[3-(N¹-morpholino)propyl] carbamate (the intermediate product obtained from Example 10, Step A) (10 g), 1-octanol (15.0 g), m-xylene (25 ml) and TBD (0.2 8) was refluxed for 1 hour. The mixture was evaporated under reduced pressure giving the product as a light yellow liquid (30.4 g). NMR analysis indicated the product to be n-octyl N-[3-(N¹-morpholino)propyl] carbamate.

### Example 13

A mixture of methyl N-[3-(N¹-morpholino)propyl] carbamate (the intermediate product obtained from Example 10, Step A) (5.2 g), polyalkylene glycol (PAG B225 from Hythe Chemicals Limited) (49.5 g), TBD (0.4 g) and m-xylene (33.4 g) was stirred at 140°C with a nitrogen sparge for 2 hours. The mixture was evaporated under reduced pressure giving the product as a viscous yellow liquid (45 g).

### Example 14

A mixture of methyl N-[2-(N¹-piperazino)ethyl] carbamate (the product of Example 8) (5.8 g), 1-octanol (4.3 g) and TBD (0.6 g) was heated at 150°C for 2 hours. The mixture was evaporated under reduced pressure giving a red-brown product (8.2 g), indicated by NMR analysis to be n-octyl N-[2-(N¹-piperazino)ethyl] carbamate.

### Example 15

A mixture of methyl N-[3-(N¹-morpholino)propyl] carbamate (the intermediate product obtained from Example 10, Step A) (16.0 g), BREOX PC 1470 [(ex Hythe Chemicals Limited, prepared by reaction of p-dodecylphenol (1 mole) with butylene oxide (15 moles)] (10.35 g) and TBD (0.9 g) was stirred at 150°C with a nitrogen sparge for 4.25 hours. The mixture was then cooled and treated with toluene (50 ml) and magnesium sulphate (1.0 g) and allowed to stand. The mixture was then evaporated under reduced pressure giving a clear liquid product
Analysis: % Nitrogen = 1.55.

### PREPARATION OF BISCARBAMATE OF THE FORMULA (I)

### Example 16

Dimethylcarbonate (18.0 g) and bis (3-aminopropyl)amine (13.1 g) were mixed in a round-bottomed flask with TBD (0.05g), and allowed to stand at room temperature. After 16h the contents of the flask had solidified, and this solid was recrystallised from toluene to give white needles, 16.9g, 96%, mp 105° - 107°C. 'H and ¹³C NMR were consistent with structure (I).

### Example 17

Dimethyl carbonate (25 ml) and bis(aminopropyl)amine (25 ml) were mixed at 20°C and TBD (0.1 g) was added. A small exotherm was noted and the mixture was allowd to stand for 16 hours. The mixture was then stripped on a rotary evaporator and the solid residue recrystallised from toluene. The product was demonstrated by NMR to be bis[methyl(N-propyl)carbamate]amine.

### Example 18

### Step (A)

Ethylene diamine (6g) and dimethyl carbonate (18g) were mixed together in a round bottom flask at 20°C. TBD (0.1g) was added and the mixture allowed to stand for 24h. After this time a white solid product was collected and recrystallised from methanol to give bis (methoxycarbonyl) ethylene diamine. NMR and IR spectra were consistent with the desired compound.

### Step (B)

Bis(methoxycarbonyl)ethylene diamine (17.8g) obtained in Step (A) above and BREOX (RTM) PC 1362 (412g) (ex. Hythe Chemicals Limited) were dissolved in A260 (a mixed aromatics solvent ex BP Chemicals Limited) (0.5 litres) and heated to 160°C with vigorous overhead stirring for 24 hours in the presence of tetraisopropyl titanate (5g). A continuous stream of dry nitrogen was passed over the reactants.

The resulting straw yellow liquid was cooled and filtered through a sinter. Its nitrogen content was determined as 0.25%.

### Example 19

The procedure of Example 18 was repeated except that the BREOX PC 1362 was replaced by PAG B225 (360g).

The nitrogen content of the product was determined as 0.37%.

### Example 20

The procedure of Example 18 was repeated except that the BREOX PC 1362 was replaced by PAG B335 (480g).

The nitrogen content of the product was determined as 0.16%.

### ENGINE TESTING

### Examples 21 to 24

The products of Examples 10, 11, 18 and 19 were evaluated in the Opel Kadett gasoline detergency test at various concentrations.

The Opel Kadett gasoline detergency test is a well-known industry accepted evaluation procedure approved by the Co-ordinating European Council (C.E.C.), Reference No. C.E.C.F.-02-T-79.

The concentrations and test results are given in the following Table.

### Comparison Test 3

The procedure of Examples 21 to 24 was repeated using instead of the biscarbamate products of Examples 10, 11, 18 and 19 a commercially available gasoline detergent having a nitrogen content of 0.70%.

The test results are given in the following Table.

**Table**

| Example | Concentration of additive (ppmw/w) | DETERMINATION* | |
|---|---|---|---|
| | | Valve Deposit Wt (mg) | Valve Deposit Rating |
| 21 | (Ex 10) 233 | 296 (409) | 7.90 (7.0) |
| 22 | (Ex 11) 320 | 110 (291) | 7.75 (7.42) |
| 23 | (Ex 18) 500 | 235 (260) | 7.32 (7.02) |
| 24 | (Ex 19) 500 | 181 (365) | 7.75 (6.62) |
| CT 3 | ( - ) 500 | 269 (440) | 7.2 (6.2) |

| | | | |
|---|---|---|---|
| * base fuel figures in parentheses. | | | |

It can be seen from the results presented in the Table that the products of Examples 10 and 11 compare favourably as detergents with the commercial product and that the products of Examples 18 and 19 have moderate detergent activity.

## Claims

1. A process for the production of a carbamate having the formula:- 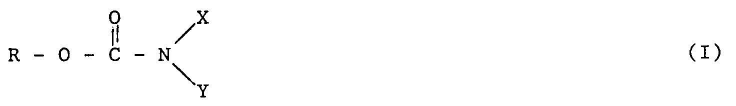 where in X and Y are independently either hydrogen, a hydrocarbyl group or a hetero-substituted hydrocarbyl group or the group of formula:- 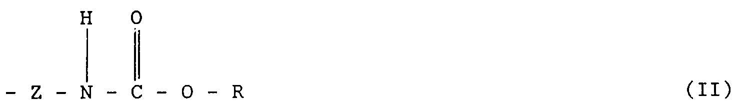 wherein Z is either a divalent hydrocarbyl, a substituted hydrocarbyl group or the group of formula:-
[ (alkylene)ₘ(NH)ₙ(alkylene)ₘ ] (III)
in which n = 0 to 4 and m = 1 to 4, and R is a hydrocarbyl or substituted hydrocarbyl group, provided that if either one of X or Y is the group of formula (II), the other of X or Y is hydrogen,
which process comprises reacting a compound of the formula:- 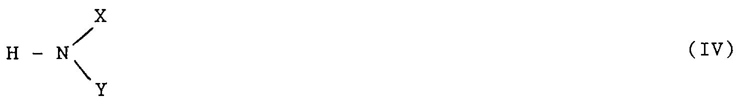 wherein X and Y are independently either hydrogen, a hydrocarbyl group or a hetero-substituted hydrocarbyl group
with a dihydrocarbyl carbonate having the formula:- 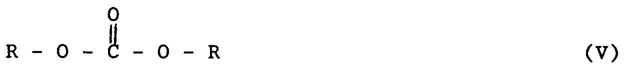 wherein independently R is as defined above
in the presence as catalyst of a strong organic base which is an amidine or a Lewis base and an epoxide and in the proportions necessary to produce either a mono-carbamate or a bis-carbamate.

2. A process according to claim 1 wherein the compound of formula (IV) is either ammonia, a primary amine or a secondary amine.

3. A process according to claim 2 wherein the compound of formula (IV) is either a monoamine or a polyamine.

4. A process according to either claim 2 or claim 3 wherein X and Y in the formula (IV) is an alkyl group.

5. A process according to either claim 2 or claim 3 wherein X and Y in the formula (IV) are independently aliphatic hydrocarbyl groups substituted by nitrogen or oxygen.

6. A process according to claim 5 wherein the hetero-substituted hydrocarbyl group is a group of the formula -P-Q wherein P is an alkylene group and Q is either NH₂, OH or a heterocyclic group.

7. A process according to any one of the preceding claims wherein in the dihydrocarbyl carbonate of formula (V) the groups R are independently C₁ to C₄ alkyl groups.

8. A process according to any one of the preceding claims wherein the amidine is a cyclic amidine.

9. A process according to any one of the preceding claims wherein the compound of formula (IV) is reacted with the dihydrocarbyl carbonate of formula (V) at a temperature in the range from 0 to 100°C and a pressure in the range from 1 to 10 bar.

10. A process according to any one of the preceding claims wherein the group R in the carbamate of formula (I) is exchanged with a group R¹ by a process which comprises reacting the carbamate of formula (I) with a compound of the formula:-
R¹OH (VI)
wherein R¹ is a hydrocarbyl or hetero-substituted hydrocarbyl group different from R
in the presence as catalyst of either a strong organic base or a tetrahydrocarbyl titanate.

11. A process according to claim 10 wherein the group R¹ in the compound of formula (VI) is a hydrocarbyl or hetero-substituted hydrocarbyl group of a molecular weight and composition such as to impart fuel solubility and the product is a detergent additive to internal combustion engine fuels.

12. A process according to claim 11 wherein the compound of formula (VI) is a polyalkylene glycol formed by the reaction of a hydroxylic compound with an alkylene oxide.

13. A process according to claim 12 wherein the polyalkylene glycol has a molecular weight in the range from about 500 to 10,000.

14. A process according to claim 11 wherein the compound of formula (VI) is the polyalkylene glycol obtained by the reaction of p-dodecylphenol with butylene oxide and has a molecular weight of about 2,000.

15. A process according to claim 11 wherein the compound of formula (VI) is a polyalkylene glycol produced by the hydroxyalkylation of an amine.

16. A process according to any one of claims 10 to 15 wherein the catalyst is a strong organic base which is the same as that used in the process for the production of the compound of formula (I).

17. A process according to any one of claims 10 to 15 wherein the catalyst is a tetraalkyltitanate.

18. A process according to any one of claims 10 to 17 wherein the compound of formula (I) is reacted with the compound of formula (VI) in the presence of a hydrocarbon solvent.

19. A process according to any one of claims 10 to 18 wherein the compound of formula (I) is reacted with the compound of formula (VI) at a temperature in the range from 100 to 300°C and at either atmospheric or superatmospheric pressure.

## Patentansprüche

1. Verfahren zur Herstellung eines Carbamats der Formel: 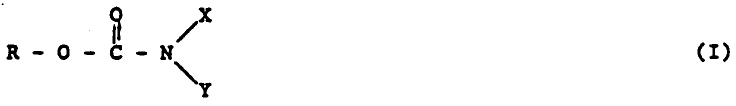 worin X und Y unabhängig voneinander für entweder Wasserstoff, eine Kohlenwasserstoffgruppe oder eine heterosubstituierte Kohlenwasserstoffgruppe oder eine Gruppe der Formel: 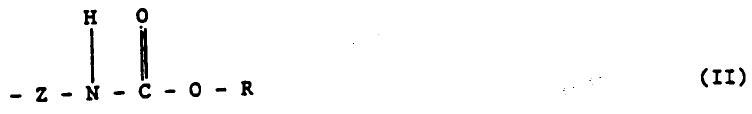 mit Z entweder gleich einer zweiwertigen Kohlenwasserstoffgruppe oder einer substituierten Kohlenwasserstoffgruppe oder einer Gruppe der Formel:
[ (Alkylen)ₘ(NH)ₙ(Alkylen)ₘ ] (III)
worin n = 0 bis 4 ist und m = 1 bis 4 ist, stehen und
R eine Kohlenwasserstoff- oder substituierte Kohlenwasserstoffgruppe ist, vorausgesetzt, daß, wenn einer der Reste X oder Y für eine Gruppe der Formel (II) steht, der andere Rest von X oder Y Wasserstoff ist,
durch Umsetzen einer Verbindung der Formel: 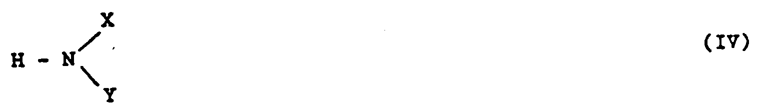 worin X und Y unabhängig voneinander für Wasserstoff, eine Kohlenwasserstoffgruppe oder eine heterosubstituierte Kohlenwasserstoffgruppe stehen,
mit einem zwei Kohlenwasserstoffsubstituenten aufweisenden Carbonat der Formel: 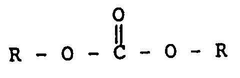 worin unabhängig voneinander R die oben angegebene Bedeutung aufweist, in Gegenwart einer starken organischen Base, bei der es sich um ein Amidin handelt oder einer Lewis-Base und eines Epoxids als Katalysator und in den zur Bildung entweder eines Monocarbamats oder eines Biscarbamats notwendigen Anteilen.

2. Verfahren nach Anspruch 1, wobei die Verbindung der Formel (IV) entweder Ammoniak, ein primäres Amin oder ein sekundäres Amin ist.

3. Verfahren nach Anspruch 2, wobei die Verbindung der Formel (IV) entweder ein Monoamin oder ein Polyamin ist.

4. Verfahren nach Anspruch 2 oder 3, wobei X und Y in der Formel (IV) für eine Alkylgruppe stehen.

5. Verfahren nach Anspruch 2 oder 3, wobei X und Y in der Formel (IV) unabhängig voneinander für aliphatische Kohlenwasserstoffgruppen, die durch Stickstoff oder Sauerstoff substituiert sind, stehen.

6. Verfahren nach Anspruch 5, wobei die heterosubstituierte Kohlenwasserstoffgruppe eine Gruppe der Formel -P-Q mit P gleich einer Alkylengruppe und Q entweder gleich NH₂, OH oder einer heterocyclischen Gruppe ist.

7. Verfahren nach einem der vorhergehenden Ansprüche, wobei in dem zwei Kohlenwasserstoffsubstituenten aufweisenden Carbonat der Formel (V) die Gruppen R unabhängig voneinander für C₁-C₄-Alkylgruppen stehen.

8. Verfahren nach einem der vorhergehenden Ansprüche, wobei das Amidin ein cyclisches Amidin ist.

9. Verfahren nach einem der vorhergehenden Ansprüche, wobei die Verbindung der Formel (IV) mit dem zwei Kohlenwasserstoffsubstituenten aufweisenden Carbonat der Formel (V) bei einer Temperatur im Bereich im Bereich von 0 bis 100°C und einem Druck im Bereich von 1 bis 10 bar umgesetzt wird.

10. Verfahren nach einem der vorhergehenden Ansprüche, wobei die Gruppe R in dem Carbamat der Formel (I) durch eine Gruppe R¹ im Rahmen eines Verfahrens ausgetauscht wird, das ein Umsetzen des Carbamats der Formel (I) mit einer Verbindung der Formel:
R¹OH (VI)
worin R¹ für eine Kohlenwasserstoff- oder heterosubstituierte Kohlenwasserstoffgruppe, die von R verschieden ist, steht,
in Gegenwart entweder einer starken organischen Base oder eines vier Kohlenwasserstoffsubstituenten aufweisenden Titanats als Katalysator umfaßt.

11. Verfahren nach Anspruch 10, wobei die Gruppe R¹ in der Verbindung der Formel (VI) eine Kohlenwasserstoff- oder heterosubstituierte Kohlenwasserstoffgruppe eines derartigen Molekulargewichts und einer derartigen Zusammensetzung ist, daß eine Kraftstofflöslichkeit erreicht wird und das Produkt ein Detergensadditiv für Verbrennungsmotorkraftstoffe ist.

12. Verfahren nach Anspruch 11, wobei die Verbindung der Formel (VI) ein durch Umsetzung einer Hydroxyverbindung mit einem Alkylenoxid gebildetes Polyalkylenglykol ist.

13. Verfahren nach Anspruch 12, wobei das Polyalkylenglykol ein Molekulargewicht im Bereich von etwa 500 bis 10.000 aufweist.

14. Verfahren nach Anspruch 11, wobei die Verbindung der Formel (VI) ein durch Umsetzung von p-Dodecylphenol mit Butylenoxid erhaltenes Polyalkylenglykol eines Molekulargewichts von etwa 2000 ist.

15. Verfahren nach Anspruch 11, wobei die Verbindung der Formel (VI) ein durch Hydroalkylierung eines Amins gebildetes Polyalkylenglykol ist.

16. Verfahren nach einem der Ansprüche 10 bis 15, wobei der Katalysator eine starke organische Base ist, die derjenigen entspricht, die im Verfahren zur Herstellung der Verbindung der Formel (I) verwendet wird.

17. Verfahren nach einem der Ansprüche 10 bis 15, wobei der Katalysator ein Tetraalkyltitanat ist.

18. Verfahren nach einem der Ansprüche 10 bis 17, wobei die Verbindung der Formel (I) in Gegenwart eines Kohlenwasserstofflösungsmittels mit der Verbindung der Formel (VI) umgesetzt wird.

19. Verfahren nach einem der Ansprüche 10 bis 18, wobei die Verbindung der Formel (I) bei einer Temperatur im Bereich von 100 bis 300°C und bei entweder Atmosphärendruck oder einem Druck über Atmosphärendruck mit der Verbindung der Formel (VI) umgesetzt wird.

## Revendications

1. Procédé pour la production d'un carbamate ayant la formule 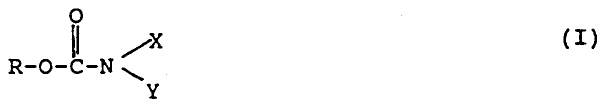 dans laquelle X et Y sont indépendamment l'un de l'autre soit un atome d'hydrogène, un groupe hydrocarbyle ou un groupe hydrocarbyle hétérosubstitué, soit le groupe de formule 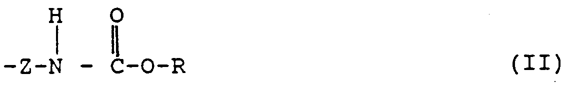 dans laquelle
Z est soit un groupe hydrocarbyle divalent ou un groupe hydrocarbyle substitué soit un groupe de formule
[(alkylène)ₘ(NH)ₙ(alkylène)ₘ] (III)
dans laquelle n = 0 à 4 et m = 1 à 4, et R est un groupe hydrocarbyle ou hydrocarbyle substitué, étant entendu que si l'un de X ou Y est le groupe de formule (II), l'autre X ou Y est un atome d'hydrogène, ledit procédé comprend la réaction d'un composé de formule: 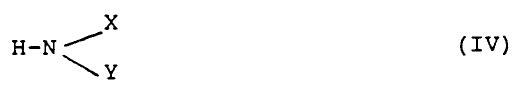 dans laquelle X et Y sont indépendamment l'un de l'autre soit un atome d'hydrogène, un groupe hydrocarbyle, soit un groupe hydrocarbyle hétérosubstitué
avec un carbonate de dihydrocarbyle ayant la formule: 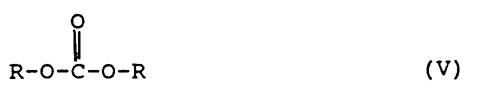 dans laquelle R est indépendamment tel que défini ci-dessus en présence, en tant que catalyseur, d'une base organique forte qui est une amidine ou d'une base de Lewis avec un époxyde et dans les proportions nécessaires pour produire soit un monocarbamate soit un biscarbamate.

2. Procédé selon la revendication 1, dans lequel le composé de formule (IV) est l'ammoniac, une amine primaire, ou une amine secondaire.

3. Procédé selon la revendication 2, dans lequel le composé de formule (IV) est soit une monoamine soit une polyamine.

4. Procédé selon l'une des revendications 2 ou 3, dans lequel X et Y dans la formule (IV) sont un groupe alkyle.

5. Procédé selon l'une des revendications 2 ou 3, dans lequel X et Y dans la formule (IV) sont indépendamment l'un de l'autre des groupes aliphatiques hydrocarbyle substitués par un atome d'azote ou d'oxygène.

6. Procédé selon la revendication 5, dans lequel le groupe hydrocarbyle hétérosubstitué est un groupe de formule -P-Q dans lequel P est un groupe alkylène et Q est NH₂, OH ou un groupe hétérocyclique.

7. Procédé selon l'une des revendications précédentes, dans lequel, dans le carbonate de dihydrocarbyle de formule (V), les groupes R sont indépendamment les uns des autres des groupes alkyle en C₁ à C₄.

8. Procédé selon l'une des revendications précédentes, dans lequel l'amidine est une amidine cyclique.

9. Procédé selon l'une des revendications précédentes dans lequel on fait réagir le composé de formule (IV) avec le carbonate de dihydrocarbyle de formule (V) à une température comprise entre 0 et 100°C et sous une pression comprise entre 0,1 et 1 MPa (1 et 10 bars).

10. Procédé selon l'une des revendications précédentes dans lequel le groupe R dans le carbamate de formule (I) est remplacé par un groupe R¹ à l'aide d'un procédé qui comprend la réaction du carbamate de formule (I) avec un composé de la formule:
R¹OH (VI)
dans laquelle R¹ est un groupe hydrocarbyle ou un groupe hydrocarbyle hétérosubstitué différent de R, en présence, en tant que catalyseur, soit d'une base organique forte soit d'un titanate de tétrahydrocarbyle.

11. Procédé selon la revendication 10, dans lequel le groupe R¹ dans le composé de formule (VI) est un groupe hydrocarbyle ou un groupe hydrocarbyle hétérosubstitué de masse moléculaire et de composition telles qu'elles confèrent au produit obtenu une solubilité dans un carburant et le produit est un additif de détergence pour carburants de moteur à combustion interne.

12. Procédé selon la revendication 11, dans lequel le composé de formule (VI) est un polyalkylèneglycol formé par la réaction d'un composé hydroxyle avec un oxyde d'alkylène.

13. Procédé selon la revendication 12, dans lequel le polyalkylèneglycol a une masse moléculaire comprise entre 500 et 10 000.

14. Procédé selon la revendication 11, dans lequel le composé de formule (VI) est le polyalkylèneglycol obtenu par réaction de p-dodécylphénol avec de l'oxyde de butylène et possède une masse moléculaire d'environ 2 000.

15. Procédé selon la revendication 11, dans lequel le composé de formule (VI) est un polyalkylèneglycol produit par l'hydroxyalkylation d'une amine.

16. Procédé selon l'une des revendications 10 à 15, dans lequel le catalyseur est une base organique forte qui est la même que celle utilisée dans le procédé pour la production du composé de formule (I).

17. Procédé selon l'une des revendications 10 à 15, dans lequel le catalyseur est un titanate de tétraalkyle.

18. Procédé selon l'une des revendications 10 à 17, dans lequel on fait réagir le composé de formule (I) avec le composé de formule (VI) en présence d'un solvant hydrocarboné.

19. Procédé selon l'une des revendications 10 à 18, dans lequel on fait réagir le composé de formule (I) avec le composé de formule (VI) à une température comprise entre 100 et 300°C et sous une pression supérieure ou égale à la pression atmosphérique.
